# EUROPEAN PATENT APPLICATION

(11) **EP 2 238 991 A2**
(43) Date of publication of application: **13.10.2010**
(21) Application number: 10250737.3
(22) Date of filing: 08.04.2010
(51) Int. Cl.: A61L 15/44, A61L 15/58

(54) **Patch and patch preparation**

(30) Priority: 10.04.2009 JP 2009005994
(71) Applicant: Nitto Denko Corporation, Ibaraki-shi, Osaka 567-8680 (JP)
(72) Inventor: Makabe, Miki, Ibaraki-shi Osaka 567-8680 (JP); Kasahara, Tsuyoshi, Ibaraki-shi Osaka 567-8680 (JP); Hamada, Atsushi, Ibaraki-shi Osaka 567-8680 (JP); Ishikura, Jun, Ibaraki-shi Osaka 567-8680 (JP)
(74) Representative: Duckworth, Timothy John

(57) **Abstract**

Provided are a patch and a patch preparation having an adhesive layer on at least one surface of a support, and particularly, a patch and a patch preparation superior in skin adhesive force, and causing less adhesive residue on the skin surface. A patch having a support and an adhesive layer formed on at least one surface of the support, wherein the adhesive layer is obtained by crosslinking a first polymer having a butadiene skeleton portion and a second polymer different from the first polymer in the presence of an organic peroxide.

## Description

### TECHNICAL FIELD OF THE INVENTION

The present invention relates to a patch and a patch preparation having an adhesive layer on at least one surface of a support, and particularly relates to a patch and a patch preparation superior in skin adhesive force, and causing less adhesive residue on the skin surface.

### BACKGROUND OF THE INVENTION

Patch is a convenient and effective dosage form for wound protection or administration of a drug to living organisms. Patch is requested to achieve improved adhesive property while stably maintaining the quality for a long time, such as enhanced skin adhesive force, reduced adhesive residue on the skin surface and the like.
Heretofore, as a patch working on the achievement of good adhesive property while stably maintaining the quality of the patch for a long time, such as enhanced skin adhesive force, reduced adhesive residue on the skin surface and the like, a patch having two or more rubber adhesives in combination is disclosed (patent document 1).
However, the invention described in patent document 1 does not disclose a polymer having a butadiene skeleton portion, and does not describe or suggest crosslinking in the presence of an organic peroxide. Moreover, when such patch is stored for a long time, the adhesive layer may develop what is called a cold flow (phenomenon of plastic deformation at a storage temperature).
[prior art reference]
[patent document]

patent document 1: W02001/043729

### [SUMMARY OF THE INVENTION]

### Problems to be Solved by the Invention

The present invention has been made in view of the above-mentioned situation, and aims to solve the problem of provision of a patch with improved adhesive property such as enhanced skin adhesive force, reduced adhesive residue on the skin surface and the like, which maintains the quality of patch stably for a long time.

### Means of Solving the Problems

The present inventors have conducted intensive studies and found that an adhesive layer containing an elastomer obtained by crosslinking a particular polymer in the presence of an organic peroxide shows superior practicality based on its improved adhesive property such as enhanced skin adhesive force, reduced adhesive residue on the skin surface and the like, which resulted in the completion of the present invention. Accordingly, the present invention provides the following.
[1] A patch comprising a support and an adhesive layer formed on at least one surface of the support, wherein the aforementioned adhesive layer is obtained by crosslinking a first polymer having a butadiene skeleton portion and a second polymer different from the aforementioned first polymer in the presence of an organic peroxide.
[2] The patch of the above-mentioned [1], wherein the aforementioned first polymer is polybutadiene.
[3] The patch of the above-mentioned [1] or [2], wherein the aforementioned second polymer is at least one kind selected from the group consisting of a copolymer having a styrene unit and an isoprene unit, polyisobutylene, polyisoprene, and a copolymer comprising a styrene unit and a butadiene unit.
[4] The patch of any of the above-mentioned [1] to [3], wherein the aforementioned organic peroxide is dibenzoyl peroxide.
[5] The patch of any of the above-mentioned [1] to [4], wherein the aforementioned adhesive layer further comprises a tackifier.
[6] The patch of any of the above-mentioned [1] to [5], wherein the aforementioned adhesive layer further comprises an organic liquid component.
[7] The patch of the above-mentioned [6], wherein the aforementioned organic liquid component is isopropyl myristate.
[8] A patch preparation comprising the patch of any of the above-mentioned [1] to [7], wherein the adhesive layer contains a drug.

### Effect of the Invention

Since the adhesive layer in the present invention exhibits strong tackiness, a patch that does not fall off easily in the early stages of adhesion can be obtained. When plural kinds of polymers are used, the tackiness can be synergistically enhanced. According to the present invention, moreover, since the adhesive force can also be sufficiently ensured, the patch can easily follow the movement of the skin, and a patch with improved adhesive property such as enhanced skin adhesive force, reduced adhesive residue on the skin surface and the like, which maintains the quality of patch stably for a long time can be obtained.

### [Embodiment of the Invention]

While preferable embodiments of the present invention are shown in the following, the detailed explanation and Examples are solely for illustration purposes, and do not limit the scope of the present invention.

The patch of the present invention comprises a support and an adhesive layer formed on at least one surface of the support, wherein the aforementioned adhesive layer is obtained by crosslinking a first polymer having a butadiene skeleton portion and a second polymer different from the aforementioned first polymer in the presence of an organic peroxide.

Since at least the first polymer is crosslinked, and the second polymer is also crosslinked depending on the kind thereof, the adhesive layer contains an elastomer having a hydrocarbon chain with a comparatively high molecular weight, which affords adhesive force and cohesive force preferable for a patch.
Moreover, since the first polymer used in the present invention has a butadiene skeleton portion, introduction of a crosslinkable functional group is not necessary, which enables long-term maintenance of stable quality of a patch during production and storage. Furthermore, since introduction of a functional group into the first polymer is not necessary, various first polymers can be used, and the degree of freedom of selection of the first polymer can be increased.

In the present invention, the first polymer preferably has a hydrocarbon chain moiety from the aspects of stability. When a polymer having a hydrocarbon chain moiety is used, since an elastomer formed by crosslinking has a hydrocarbon chain moiety with a comparatively high molecular weight, and a structure wherein hydrocarbon chains are three-dimensionally entangled intrinsically, the skin adhesive force and cohesive force necessary as a patch can be imparted to the adhesive layer. As the hydrocarbon chain, a nonaromatic hydrocarbon chain and an aromatic hydrocarbon chain can be mentioned, as the nonaromatic hydrocarbon chain, alkylene, alkenylene, alkynylene and the like can be mentioned, and as the aromatic hydrocarbon chain, phenylene, naphthylene and the like can be mentioned. The hydrocarbon chain only needs to be present in at least one of the main chain and the side chain.

As the first polymer, polybutadiene, styrene-butadiene block copolymer (hereinafter sometimes to be abbreviated as "SB"), styrene-butadiene-styrene block copolymer (hereinafter sometimes to be abbreviated as "SBS") and the like are preferable. When SB or SBS is used as the first polymer, a strong cohesive force can be imparted to the adhesive layer. From the aspects of reactivity of crosslinking and the like, polybutadiene is particularly preferable. While the amount of the first polymer to be used is not particularly limited, it is generally 10 - 75 wt%, preferably 20 - 50 wt%, of the solid content of the adhesive. In the present invention, one or more kinds of such first polymers can be used in combination depending on the object.

In the present specification, the second polymer refers to a polymer having a structure different from that of the first polymer. The technical significance of the difference between the second polymer and the first polymer is that a three-dimensional crosslinked structure unobtainable when they have the same structure can be obtained. It is assumed that a three-dimensional crosslinked structure obtained by crosslinking the first polymer and the second polymer having different structures imparts an adhesive layer with sufficient cohesive force and adhesive force.

While the second polymer is not particularly limited as long as it has a structure different from that of the first polymer, a copolymer having a styrene unit and an isoprene unit (e.g., styrene-isoprene-styrene copolymer), polyisobutylene, polyisoprene, and a copolymer having a styrene unit and a butadiene unit (e.g., styrene-butadiene-styrene copolymer) and the like can be mentioned. To synergistically increase the tackiness of an adhesive layer, a styrene-isoprene-styrene copolymer (hereinafter sometimes to be abbreviated as "SIS"), polyisobutylene and polyisoprene are preferable. They can be used alone or in a combination of two or more kinds thereof.

Here, the second polymer "having a structure different from that of the first polymer" includes a polymer containing a copolymerization unit different from that of the first polymer, and when the copolymerization mode thereof is different from that of the first polymer. For example, the former includes the first polymer being polybutadiene and the second polymer being SB or SBS. In addition, the former includes the first polymer being SBS and the second polymer being SIS. On the other hand, the latter includes different polymerization modes such as the first polymer being styrenebutadiene rubber (SBR) and the second polymer being SBS block copolymer.

While the weight average molecular weights of the first polymer and the second polymer are not particularly limited, they are each preferably 50,000 - 5,000,000, more preferably 100,000 - 500,000. Here, the "weight average molecular weight" is measured by gel permeation chromatography under the following conditions.

### (Analysis conditions)

GPC apparatus: HLC8120 (manufactured by Tosoh Corporation)
column: TSK gel GMH-H(S) (manufactured by Tosoh Corporation)
standard: polystyrene
eluent: tetrahydrofuran
flow rate: 0.5 mL/min
measurement temperature: 40°C
detector: differential refractometer

The amount of the second polymer relative to 100 parts by weight of the first polymer is preferably 10 - 600 parts by weight. When it exceeds 600 parts by weight, the first polymer is insufficiently crosslinked, and the cohesive force may become insufficient. When it is below 10 parts by weight, crosslinking may be too promoted to achieve necessary adhesive force.

A preferable embodiment of the present invention is characterized by crosslinking a first polymer having a butadiene skeleton portion in the presence of an organic peroxide to give an adhesive layer. The organic peroxide is not particularly limited, and known organic peroxide generally used in the polymer chemical field can be used. For example, diacyl peroxide (e.g., dibenzoyl peroxide, diisobutyryl peroxide, di(3,5,5-trimethylhexanoyl)peroxide, dilauroyl peroxide, disuccinate peroxide, peroxyester (e.g., 1,1,3,3-tetramethylbutyl peroxy-2-ethylhexanoate, 2,5-dimethyl-2,5-di(2-ethylhexanoylperoxy)hexane), t-hexylperoxy-2-ethylhexanoate, t-butyl peroxy-2-ethylhexanoate), ketone peroxide (e.g., methylethylketone peroxide), peroxy ketal (e.g., 1,1-di(t-butylperoxy)cyclohexane), hydroperoxide (e.g., p-menthane hydroperoxide), dialkyl peroxide (e.g., dicumyl peroxide), peroxy dicarbonate (e.g., di-n-propyl peroxy dicarbonate) and the like can be mentioned.

Among these, from the aspects of reactivity, diacyl peroxide (particularly dibenzoyl peroxide) and peroxyester (particularly 1,1,3,3-tetramethylbutylperoxy-2-ethylhexanoate) are preferable. Particularly, diacyl peroxide is preferable, which reacts with the first polymer to give an elastomer having an organic group such as alkyl, phenyl, acyl, benzoyl, acyloxy or benzoyloxy, each of which is optionally substituted. In addition, peroxyester reacts with the first polymer to give an elastomer having an organic group such as alkyl, alkoxy, phenyl, acyl, benzoyl, acyloxy or benzoyloxy, each of which is optionally substituted.

The amount of such organic peroxide is preferably 0.01 - 1.0 wt% relative to 100 wt% of the first polymer. When it is less than 0.01 wt%, the cohesive force of the adhesive layer tends to be insufficient, and when it exceeds 1.0 wt%, the adhesive layer tends to become hard, resulting in lower adhesive force and decreased soft feeling.

In the present invention, other components may be added to an adhesive solution to obtain an adhesive layer containing other components. Examples of other components to be contained include a tackifier as an adhesiveness imparting agent. When a tackifier is added, the first polymer is crosslinked in the presence of an organic peroxide, and takes the tackifier into its structure during formation of a three-dimensional net molecular structure as an elastomer. As a result, a large amount of a tackifier can be contained in the adhesive layer, which in turn is considered to improve the balance of adhesive force and cohesive force. Consequently, the patch of the present invention can improve adhesive force and tackiness during adhesion to the skin and reduce adhesive residue on the skin surface.

As the tackifier, one known in the field of patch can be appropriately selected for use. Examples of the tackifier include petroleum resin (e.g., aromatic petroleum resin, aliphatic petroleum resin), terpene resin, rosin resin, coumarone indene resin, styrene resin (e.g., styrene resin, α-methylstyrene), hydrogenated petroleum resin (e.g., alicyclic saturated hydrocarbon resin) and the like. Among these, an alicyclic saturated hydrocarbon resin is preferable since it improves preservation stability of other compounds in the adhesive layer, for example, an organic liquid component, a drug and the like.

Tackifier may be a combination of one or more kinds thereof. When two or more kinds are combined for use, for example, resins different in the kind and softening point may be combined.
In the present invention, the ratio ((a):(b)) of (a) the total weight of the first polymer and the second polymer and (b) the weight of the tackifier is not particularly limited. It is preferably 3.0:1 - 1:2.0, more preferably 2.5:1 - 1:1.75. When the ratio of (a) the total weight of the first polymer and the second polymer is higher than this ratio, the adhesive force of the adhesive layer tends to decrease, the cohesive force tends to be too strong, and a soft feeling tends to decrease. When the ratio of (b) the weight of the tackifier is higher than this ratio, the adhesive layer tends to be too soft and becomes sticky.

In addition, as other component to be contained, an adhesive layer may contain an organic liquid component. The effect of containing an organic liquid component is that a soft feeling is afforded during adhesion to the skin, irritation during detaching from the skin is reduced, and adhesive residue on the skin surface is reduced. Moreover, when the below-mentioned drug is contained in an adhesive layer, its transdermal absorption can be promoted.

As such organic liquid component, a hydrophobic liquid component such as fatty acid alkylester is preferable in view of compatibility with an adhesive layer. Examples of the fatty acid alkylester include fatty acid alkylester composed of higher fatty acid having a carbon number of 12 - 16, preferably 12 - 14, and monovalent lower alcohol having a carbon number of 1 - 4. As the above-mentioned higher fatty acid, lauric acid (C12), myristic acid (C14) and palmitic acid (C16) can be mentioned, with preference given to myristic acid. As the above-mentioned monovalent alcohol, methyl alcohol, ethyl alcohol, propyl alcohol, butyl alcohol and the like can be mentioned, with preference given to isopropyl alcohol. Thus, preferable fatty acid alkylester is isopropyl myristate. One or more kinds of organic liquid components may be used in combination.

The content of the organic liquid component is preferably 5 - 300 parts by weight, more preferably 50 - 170 parts by weight, relative to 100 parts by weight of the total weight of the first polymer and the second polymer. When the amount of the organic liquid component is not less than 5 parts by weight, a high soft feeling can be imparted to the adhesive layer and, when the adhesive layer contains a drug, a high transdermal absorption promoting effect can be obtained. When it is not less than 50 parts by weight, a superior soft feeling can be imparted to the adhesive layer, while suppressing a decrease in the adhesive force and cohesive force of the whole adhesive layer and, when the adhesive layer contains a drug, high transdermal absorbability is advantageously obtained.

In the present invention, moreover, a drug may be contained in an adhesive layer and a patch preparation can also be produced. As mentioned above, the first polymer does not require a functional group for crosslinking. Therefore, undesirable reaction in the adhesive layer can be suppressed, thus contributing to the stability of the drug. In addition, an undesired reaction between a drug and the functional group of the first polymer does not need to be considered, thus increasing the degree of freedom of the design of the patch.

The drug is not particularly limited, and a drug that can be administered to mammals such as human and the like through the skin thereof, i.e., a transdermally absorbable drug, is preferable. Two or more kinds of drugs can be used in combination as necessary. Specific examples of such a drug include anesthetics, hypnotic sedatives, antiepileptic drugs, antipyretic analgesic antiphlogistic drugs, anti-vertiginous drugs, psychoneurotic drugs, topical anesthetics, skeletal muscle relaxants, autonomic drugs, antiepileptic drugs, anti-Parkinsonian drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for mattery diseases, analgesic-antipruritic-styptic-antiinflammatory agent drugs, drugs for parasitic dermatic diseases, drugs for arrest of bleeding, gout treatment drugs, drugs for diabetes, anti-malignant tumor drugs, antibiotic, chemical therapy drugs, narcotic, quit smoking aids, and the like.
While the content of the drug is not particularly limited as long as the effect of the transdermally absorbable drug is provided and the adhesive property of the adhesive is not impaired, it is preferably, for example, 0.5 - 50 parts by weight relative to the total weight of 60 parts by weight of the first polymer and the second polymer.

The adhesive layer may additionally contain additives known per se, for example, anti-aging agent, antioxidant, UV absorber, filler and the like as necessary.

While the support to be used in the present invention is not particularly limited, a support substantially impermeable to the adhesive layer components such as adhesive, additive, drug and the like, namely, one that does not permit them to penetrate the support and be lost from the back face of thereof to decrease the content is preferable.

As such support, for example, a single film or a laminate film of polyester, nylon, saran (registered trade mark), polyethylene, polypropylene, polyvinyl chloride, ethylene-ethyl acrylate copolymer, ethylene-vinyl acetate copolymer, polytetrafluoroethylene, surlyn (registered trade mark), film made from ionomer resin etc., metal foil and the like, and the like can be used.

To improve the adhesive force (anchor force) between the support and the adhesive layer, the support is preferably a laminate film of a non-porous plastic film and a porous film, both composed of the above-mentioned materials, and the adhesive layer is preferably formed on the porous film side.

To improve anchor force, flexibility of the whole patch preparation and operability during adhesion, the porous film has a thickness of 10 - 200 µm. In the case of a thin preparation, a porous film of a plaster type or adhesive tape type having a thickness of 10 - 100 µm is employed. When a woven fabric or non-woven fabric is used as a porous film, the fabric weight is not subject to any particular limitation but is generally 5 - 30 g/m², preferably 6 - 15 g/m².

A most desirable support in the present invention is a laminate film of a polyester film with 1.5 - 6 µm thickness (preferably, poly(ethylene terephthalate) film) and a non-woven fabric made of polyester (preferably, poly(ethylene terephthalate)) having a basis weight of 6 - 12 g/m².

Since the patch and patch preparation of the present invention protect the adhesive face of an adhesive layer before use, a release liner is preferably laminated on the adhesive face. The release liner is not particularly limited as long as it is release-treated and ensures sufficient easy-to-release property. For example, films of polyester, polyvinyl chloride, polyvinylidene chloride, poly(ethylene terephthalate) and the like, papers such as quality paper, glassine paper and the like, or a laminate film of quality paper, glassine paper etc. and polyolefin, and the like after a detach treatment by applying silicone resin, fluorine resin and the like to the surface to be in contact with the adhesive layer can be used. Preferred is polyester. The thickness of the release sheet is generally 10 - 200 µm, preferably 25 - 100 µm.

The patch and patch preparation of the present invention can be used by detaching a release liner immediately before use and adhering the exposed adhesive face to the skin surface and the like.

The patch and patch preparation of the present invention are produced, for example, in the following manner. While the production method is not particularly limited, it includes, for example, (i) a step of dissolving or dispersing a polymer having a butadiene skeleton as a the first polymer, a second polymer and, where necessary, an adhesiveness imparting agent, an organic liquid component, a drug and the like in a solvent, (ii) a step of applying the obtained viscous solution or dispersion on at least one surface of a support, and drying same to form an adhesive layer on the surface of the support, and (iii) a step of forming a release liner on the adhesive layer (what is called direct transfer method). Alternatively, a patch and a patch preparation can also be produced by a method including (i) a step of applying the above-mentioned solution or dispersion on at least one surface of a release liner for protection, (ii) a step of drying same to form an adhesive layer on the surface of the release liner, and (iii) a step of adhering a support to the adhesive layer (what is called transcription method).

To promote crosslinking of the first polymer in an adhesive layer, the above-mentioned method preferably further includes, for example, a curing step. The curing step can be performed by heating and storing at, for example, 50 - 300°C for 10 - 100 hr. More preferably, the above-mentioned rate can be effectively controlled by performing the above-mentioned curing step in an atmosphere of oxygen concentration 0.01 - 1 vol%.

The thickness of the adhesive layer of the present invention is preferably 10 µm - 1000 µm, particularly preferably 20 µm - 500 µm, from the aspects of skin adhesiveness.

The form of the patch and patch preparation of the present invention is not particularly limited and may be, for example, a tape, a sheet, a reservoir type and the like.

The present invention is explained in more detail in the following by referring to Examples, which are not to be construed as limitative.

### Examples

### Production Example (patch)

The following Table 1 shows the content ratio of the adhesives in Examples 1 - 4 and Comparative Examples 1 - 5.

The starting materials and abbreviations thereof used in the Examples and Comparative Examples are as follows:

### <Starting materials>

BR: polybutadiene rubber, weight average molecular weight 458,000
SIS: styrene-isoprene-styrene rubber, weight average molecular weight 211,000
PIB: polyisobutylene rubber, weight average molecular weight 308,000
IR: polyisoprene rubber, weight average molecular weight 149,000
SBR: styrene butadiene rubber, weight average molecular weight 300,000

Under an air atmosphere, BR (25 wt%) as the first polymer and, as the second polymer, a second polymer of the kind and in the amount shown in Table 1 were mixed, alicyclic saturated hydrocarbon resin P100 (35 wt%, softening point 100.5°C, ring-and-ball method) as a tackifier and isopropyl myristate (29.9 wt%) as an organic liquid component were added to toluene, and they were mixed and stirred. Dibenzoyl peroxide was added as an organic peroxide to the obtained mixture to 0.1 wt%, and the mixture was further stirred to give an adhesive solution.
The adhesive solution was adjusted to have a solid content of the adhesive solution of 25 - 40 wt%, the adjusted adhesive solution was applied onto a polyester release liner (75 µm thick) such that the thickness after drying was 100 µm and dried to form an adhesive layer.
As a support, a lamination film of a polyester film (2 µm thick) and a polyester non-woven fabric (fabric weight 8 g/m²) was used, and the above-mentioned adhesive layer was laminated on the surface of the non-woven fabric of the support. This was heated and aged under a nitrogen atmosphere (oxygen concentration 0.1 vol%) at 80°C for 2 days to give the patches of Examples 1 - 4 and Comparative Examples 1 - 5.

### Production Example (patch preparation)

In the same manner as in Examples 1 - 4 except that 25 wt% of the first polymer was replaced by 24 wt% of the first polymer and 1 wt% of indomethacin, the patch preparations of Examples 5 - 8 were produced. From the following test results, the patch preparations of Examples 5 - 8 showed good property like the patches of Examples 1 - 4.

### [Experimental Examples]

The patches of Examples 1 - 4 and Comparative Examples 1 - 5 were evaluated based on the measurement of the following evaluation items. The evaluation results are shown in Table 1.

### (1) Adhesive force

In a room at 23°C, 60%RH, the patch was cut into a test piece (width 12 mm, length 5 cm), a release liner was removed from the test piece, and the test piece was press-bonded to a phenol resin test plate by one reciprocation of a 2 kg roller. The test piece was left standing under such environment for 30 min and the adhesive force was measured by stretching the test piece by a tensile tester at a detach angle of 180° and a detach rate of 300 mm/min. As for the failure mode, the cohesive failure was G and interface failure was K. Here, the average value of the adhesive force in Table 1 is an average of the measurement results of three test pieces, where a test piece having the value of not less than 0.5 (N/12 mm) and free of cohesive failure, i.e., failure mode is interface failure "K", is preferable as an evaluation of adhesive force. In addition, SD means standard deviation.

### (2) Ball tackiness

The tackiness of the adhesive layer was measured according to the inclination ball tack testing method (inclination angle: 30°, 23°C, 65%RH) provided in JIS Z 0237. Specifically, an adhesive layer was set at a predetermined position of an inclination ball tack apparatus (ball rolling apparatus) wherein the angle of an inclination plane had been set to 30°, and a poly(ethylene terephthalate) film (25 µm-thick poly(ethylene terephthalate) film defined in JIS C 2318) as an approach was adhered to a predetermined position of the adhesive surface. Then, a ball was placed such that the center of the ball was on the ball start position and the length of the approach was 50 mm irrespective of the size of the ball, and the ball was rolled. A ball having a maximum size was found from the balls that had stopped on the adhesive surface within the measurement area, and said maximum ball found and the balls having sizes before and after said maximum size (total 3 balls) were rolled again in the same manner as above (once for each ball, total 3 times of rolling) in an attempt to confirm that the ball found to have the maximum size was indeed the maximum ball meeting the measurement conditions. Then, the maximum ball diameter (mm) was determined and taken as the tackiness of the adhesive layer.

From the adhesive force evaluation results in Table 1, the patches of Examples 1 - 4 showed an adhesive force of not less than 0.5 (N/12 mm) and had adhesive force and cohesive force necessary for a patch. The patches of Examples 1 - 4 developed interface failure between the testing plane and the adhesive layer during the adhesive force measurement, and an adhesive residue was not found on the testing plane. In addition, higher adhesive force than in Comparative Example 5 using BR alone was obtained.
According to the failure mode evaluation results, the patches of Comparative Examples 1 - 4 showed insufficient (G) cohesive force and developed cohesive failure during the adhesive force measurement, and an adhesive residue was observed. The adhesive layers of Comparative Examples 1 - 4 contain only one kind of polymer, and the one kind of polymer contained is considered to have not been crosslinked.
The results of the ball tackiness test of the adhesive layers of Examples 1 - 4 were diameters of not less than 7.1 mm, and are good. On the other hand, in Comparative Example 1, the diameter was 4.8 mm, showing weaker tackiness than in Examples 1 - 4, and the diameter was 3.2 mm, showing weaker tackiness, even for Comparative Example 5 having cohesive force. As for Comparative Examples 2 and 3, even though the cohesive force was weak, the tackiness was 10.3 mm for both.
As explained above, it has been shown that only the adhesives of Examples 1 - 4 belonging to the present invention have required adhesive force and sufficient cohesive force, and are also superior in the ball tackiness.

**Table 1**

| | composition (wt %) | | | | | | property evaluation results | | | | |
|---|---|---|---|---|---|---|---|---|---|---|---|
| | BR (first polymer) | second polymer | | tackifier | isopropyl myristate | dibenzoyl peroxide | adhesive force (N/12 mm) | | | ball tackiness (mm) | |
| | | kind | (wt %) | | | | average | SD | failure mode | average | SD |
| Ex. 1 | 25 | SIS | 10 | 35 | 29.9 | 0.1 | 1.46 | 0.04 | K | 9.5 | 0 |
| Ex. 2 | 25 | PIB | 10 | 35 | 29.9 | 0.1 | 1.29 | 0.08 | K | 7.1 | 0 |
| Ex. 3 | 25 | IR | 10 | 35 | 29.9 | 0.1 | 1.16 | 0.05 | K | 9.5 | 0 |
| Ex. 4 | 25 | SBR | 10 | 35 | 29.9 | 0.1 | 1.75 | 0.05 | K | 7.1 | 0 |
| Com. Ex. 1 | 0 | SIS | 35 | 35 | 29.9 | 0.1 | 1.45 | 0.11 | G | 4.8 | 0 |
| Com. Ex. 2 | 0 | PIB | 35 | 35 | 29.9 | 0.1 | 1.73 | 0.04 | G | 10.3 | 0 |
| Com. Ex. 3 | 0 | IR | 35 | 35 | 29.9 | 0.1 | 1.31 | 0.02 | G | 10.3 | 0 |
| Com. Ex. 4 | 0 | SBR | 35 | 35 | 29.9 | 0.1 | - | - | G | - | - |
| Com. Ex. 5 | 0 | BR | 35 | 35 | 29.9 | 0.1 | 0.82 | 0.10 | K | 3.2 | 0 |

### INDUSTRIAL APPLICABILITY

The patch of the present invention shows good adhesion property while stably maintaining the quality for a long time, and is also useful as a patch preparation.

## Claims

1. A patch comprising a support and an adhesive layer formed on at least one surface of the support, wherein the adhesive layer is obtained by crosslinking a first polymer having a butadiene skeleton portion and a second polymer different from the first polymer in the presence of an organic peroxide.

2. The patch according to claim 1, wherein the first polymer is polybutadiene.

3. The patch according to claim 1 or 2, wherein the second polymer is at least one kind selected from the group consisting of a copolymer having a styrene unit and an isoprene unit, polyisobutylene, polyisoprene, and a copolymer comprising a styrene unit and a butadiene unit.

4. The patch according to any of claims 1 to 3, wherein the organic peroxide is dibenzoyl peroxide.

5. The patch according to any of claims 1 to 4, wherein the adhesive layer further comprises a tackifier.

6. The pitch according to any of claims 1 to 5, wherein the adhesive layer further comprises an organic liquid component.

7. The patch according to claim 6, wherein the organic liquid component is isopropyl myristate.

8. A patch preparation comprising the patch according to any of claims 1 to 7, wherein the adhesive layer contains a drug.
